# EUROPÄISCHE PATENTANMELDUNG

(11) **EP 4 763 243 A1**
(43) Veröffentlichungstag der Anmeldung: **24.06.2026**
(21) Anmeldenummer: 25222094.2
(22) Anmeldetag: 10.12.2025
(51) Int. Cl.: A61M 1/34

(54) **VORRICHTUNG ZUR EXTRAKORPORALEN BLUTBEHANDLUNG**

(30) Priorität: 17.12.2024 DE 102024138426
(71) Anmelder: B. Braun Avitum AG, 34212 Melsungen (DE)
(72) Erfinder: RIEMENSCHNEIDER, Heiko, 34327 Wagenfurth (DE); PETERS, Reinhardt, 50374 Erftstadt (DE)
(74) Vertreter: Patentanwälte Ruff, Wilhelm, Beier, Dauster & Partner mbB

(57) **Zusammenfassung**

Vorrichtung (100) zur extrakorporalen Blutbehandlung, mittels der eine Hämodialyse und eine Hämodiafiltration ausführbar ist, aufweisend:
- einen Dialysator (1),
- eine elektrische Blutpumpe (2) zum Fördern von Blut durch eine Blutseite (1a) des Dialysators (1),
- eine elektrische Substituatpumpe (4) zum Fördern von Substituat (3) und
- eine Steuereinrichtung (5), die die elektrische Blutpumpe (2) und die elektrische Substituatpumpe (4) derart ansteuert, dass während einer Hämodiafiltration
- Blut durch eine Blutseite (1a) des Dialysators (1) mittels der elektrischen Blutpumpe (2) gefördert wird,
- Substituat (3) mittels der elektrischen Substituatpumpe (4) gefördert wird, und
- ein Fördervolumenstrom der elektrischen Substituatpumpe (4) derart eingestellt wird, dass eine Stromaufnahme der elektrischen Blutpumpe (2) innerhalb vorgebbarer Grenzwerte bleibt.

## Beschreibung

Der Erfindung liegt die Aufgabe zugrunde, eine Vorrichtung zur extrakorporalen Blutbehandlung zur Verfügung zu stellen, die ein einfach und effizient betreibbar ist.

Die Vorrichtung zur extrakorporalen Blutbehandlung bzw. die Dialysemaschine weist auf: einen Dialysator, eine elektrische Blutpumpe zum Fördern von Blut durch eine Blutseite des Dialysators, eine elektrische Substituatpumpe zum Fördern von Substituat und eine Steuereinrichtung, die die elektrische Blutpumpe und die elektrische Substituatpumpe wie nachfolgend beschrieben ansteuert.

Mittels der Vorrichtung zur extrakorporalen Blutbehandlung ist eine Hämodialyse (HD) und eine Hämodiafiltration (HDF) ausführbar. Hinsichtlich der an sich bekannten Hämodialyse sei auf die einschlägige Fachliteratur verwiesen. Während der Hämodiafiltration steuert die Steuereinrichtung die elektrische Blutpumpe und die elektrische Substituatpumpe derart an, dass Blut durch eine Blutseite des Dialysators mittels der elektrischen Blutpumpe gefördert wird, Substituat mittels der elektrischen Substituatpumpe gefördert wird, und ein Fördervolumenstrom der elektrischen Substituatpumpe derart eingestellt wird, dass eine Stromaufnahme der elektrischen Blutpumpe innerhalb vorgebbarer Grenzwerte bleibt. Mit anderen Worten regelt die Steuereinrichtung die Stromaufnahme der elektrischen Blutpumpe auf einen vorgebbaren Sollwert, wobei die Stellgröße der Regelung der Fördervolumenstrom der elektrischen Substituatpumpe ist. Die Stromaufnahme der elektrischen Blutpumpe korrespondiert mit dem mittels der elektrischen Blutpumpe erzeugten Drehmoment, wobei Strom und Drehmoment im Sinne der Erfindung als äquivalent betrachtet und verwendet werden.

In einer Ausführungsform wird bei einem Ansteigen der Stromaufnahme der elektrischen Blutpumpe über einen oberen Grenzwert hinaus der Fördervolumenstrom der elektrischen Substituatpumpe reduziert. Entsprechend wird bei einem Abnehmen der Stromaufnahme der elektrischen Blutpumpe unter einen unteren Grenzwert der Fördervolumenstrom der elektrischen Substituatpumpe erhöht. Das Reduzieren bzw. das Erhöhen des Fördervolumenstroms der elektrischen Substituatpumpe kann beispielsweise proportional zu einem Betrag einer Differenz zwischen der Stromaufnahme der elektrischen Blutpumpe und dem oberen Grenzwert bzw. dem unteren Grenzwert erfolgen. Weiter ist es möglich, dass das Reduzieren bzw. das Erhöhen des Fördervolumenstroms der elektrischen Substituatpumpe um einen festen Betrag erfolgt, usw.

In einer Ausführungsform wird das Substituat mittels einer Postdilution einem Blutkreislauf eines Patienten zugeführt.

In einer Ausführungsform bewirkt die Steuereinrichtung zunächst eine Hämodialyse, dann überwacht die Steuereinrichtung fortlaufend, ob sich eine Sekundärmembran auf einer Membran des Dialysators gebildet hat, und, sobald sich die Sekundärmembran auf der Membran des Dialysators gebildet hat, schaltet die Steuereinrichtung auf die Hämodiafiltration um. Hinsichtlich des Überwachens bzw. Feststellens der Sekundärmembranbildung sei auf die relevante Fachliteratur verwiesen, beispielsweise auf die EP 3 231 464 A1.

In einer Ausführungsform stellt die Steuereinrichtung nach dem Umschalten auf die Hämodiafiltration eine vorgegebene Cross Rate ein, misst dann eine sich ergebende Stromaufnahme der elektrischen Blutpumpe bei der vorgegebenen Cross Rate und berechnet schließlich die vorgebbaren Grenzwerte abhängig von der gemessenen Stromaufnahme.

Die Cross Rate ist definiert als das Verhältnis zwischen der Ultrafiltrationsrate Quf und der Plasmaflussrate Qp, also Cross Rate = Quf/Qp. Die Ultrafiltrationsrate Quf wird vom Anwender der Dialysemaschine eingestellt und ist maßgebend für die Menge an Flüssigkeit, die dem Patienten durch die Behandlung entzogen wird. Verantwortlich für die Ultrafiltrationsrate ist die Ultrafiltrationspumpe, die die Flüssigkeit durch die Membran des Dialysators "saugt". Die Plasmaflussrate Qp entspricht der Blutflussrate der Blutpumpe, die das Blut des Patienten zum Dialysator transportiert. Eine Cross Rate von 25% bedeutet beispielsweise, dass 25% der Flüssigkeit im Blut durch die Ultrafiltration entzogen wird. Je höher die Cross Rate, desto mehr Flüssigkeit wir dem Blut entzogen und desto dicker wird das Blut, was letztendlich zu einer Verstopfung des Dialysators führen kann.

In einer Ausführungsform wird der obere Grenzwert in einem Bereich zwischen 115 % und 135 % der gemessenen Stromaufnahme vorgegeben, und/oder der untere Grenzwert wird als das 0,8-fache bis 0,95-fache des oberen Grenzwerts vorgegeben.

Erfindungsgemäß wird mittels der Vorrichtung zur extrakorporalen Blutbehandlung der Behandlungsverlauf des Dialysators automatisch über die Stromzufuhr der Blutpumpe bewertet. Die Substitutionsmenge wird bei erkennbaren Stromanstiegen der Blutpumpe automatisch reduziert. Entsprechend wird die Substitutionsmenge wieder automatisch erhöht, sobald der Strom der Blutpumpe wieder auf einen errechneten Basiswert plus x % abgefallen ist, wobei die x % Patientenspezifisch angepasst werden können.

Erfindungsgemäß erfolgt mittels der Vorrichtung zur extrakorporalen Blutbehandlung automatisch ein verzögerter HDF-Start in der Betriebsart HDF mit Postdilution. Erfindungsgemäß wird die Stromaufnahmeveränderung der arteriellen Blutpumpe gemessen und ausgewertet.

Der Strom der Blutpumpe wird am Beginn einer Therapie gemessen und als Basis dafür verwendet, einen Grenzwert beispielsweise ca. 20 % bis 30% über diesem gemessenen Anfangswert zu halten.

Wird eine beginnende Verstopfung des Dialysators durch einen korrespondierenden Stromanstieg erkannt, wird die Substitution so lange reduziert, bis der Grenzwert wieder unterschritten wird.

Zum Erkennen einer Verblockung werden die Veränderungen der Drehmomente bzw. des Motorantriebsstromes der Blutpumpe gemessen und bewertet.

Es ist möglich, einen festen Alarm-Grenzwert vorzugeben, der nicht überschritten werden darf, beispielsweise ein Motorantriebsstrom, der einem Drehmoment > 50mNm entspricht. Normal liegt dieser Wert bei ca. 20 mNm.

Des Weiteren wird ein sich einstellender Wert beispielsweise während der ersten 10 Minuten Behandlungsdauer ermittelt und eine zusätzlich Warngrenze festgelegt. Diese ist beispielsweise ca. 30 mNm höher als der ermittelte Wert der ersten 10 Minuten. Eine Warnung bzw. Alarm-Ausgabe erfolgt nur bei dauerhafter Überschreitung dieses Grenzwertes. Anlaufstrom bzw. Anlaufdrehmoment bleiben bei der Überwachung damit außerhalb der Betrachtung.

Die Erfindung verhindert Anwendungsfehler und minimiert das Risiko eines durch zu hohe Cross Raten verblockenden Dialysators während der HDF. Es ist nicht mehr notwendig darauf zu achten, wann die Sekundärmembran weit genug ausgebildet ist, da der Zeitpunkt der Umschaltung von HD auf HDF und die Substitutionsmenge automatisch berechnet werden können.

Die Vorrichtung zur extrakorporalen Blutbehandlung unterstützt einen Anwender und überwacht die Therapie automatisch. Infundierte Mengen können kontinuierlich angezeigt werden, ebenso wie zu erwartende, mit der momentanen Einstellung erreichbare Größen.

Es kann jederzeit ein patientenbezogener Grenzwert des Motostroms individuell eingestellt werden.

Die Erfindung wird nachfolgend unter Bezugnahme auf die Zeichnungen detailliert beschrieben. Hierbei zeigt:

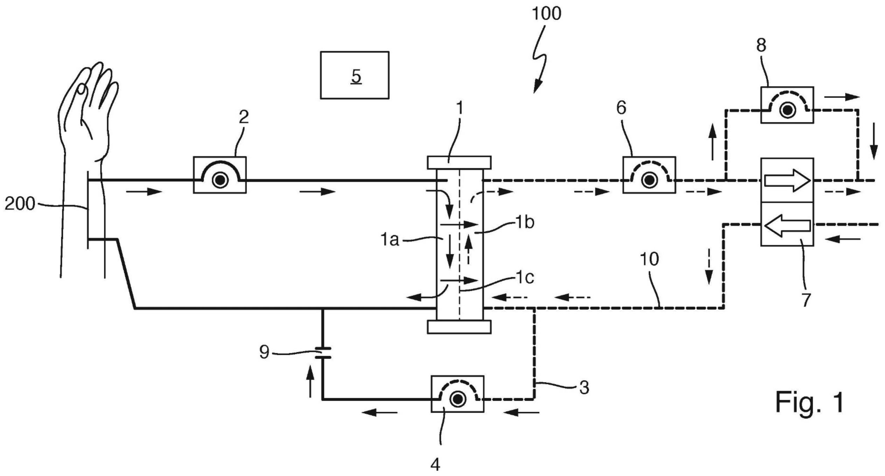

- Fig. 1: schematisch eine Vorrichtung zur extrakorporalen Blutbehandlung und
- Fig. 2: ein Flussdiagramm eines mittels einer Steuereinrichtung der Vorrichtung zur extrakorporalen Blutbehandlung ausgeführten Verfahrens.

Die Vorrichtung 100 zur extrakorporalen Blutbehandlung weist auf: einen Dialysator 1, eine elektrische Blutpumpe 2 zum Fördern von Blut durch eine Blutseite 1a des Dialysators 1, eine elektrische Substituatpumpe 4 zum Fördern von Substituat 3, eine Dialysatpumpe 6 zum Fördern von Dialysat 10 durch eine Dialysatseite 1b des Dialysators 1, eine Bilanzierungsvorrichtung 7 und eine Ultrafiltrationspumpe 8. Hinsichtlich der grundsätzlichen Funktion dieser Komponenten sei auch auf die einschlägige Fachliteratur verwiesen.

Die Vorrichtung 100 zur extrakorporalen Blutbehandlung weist weiter eine Steuereinrichtung 5 auf, die die elektrische Blutpumpe 2 und die elektrische Substituatpumpe 4 wie nachfolgend beschrieben ansteuert.

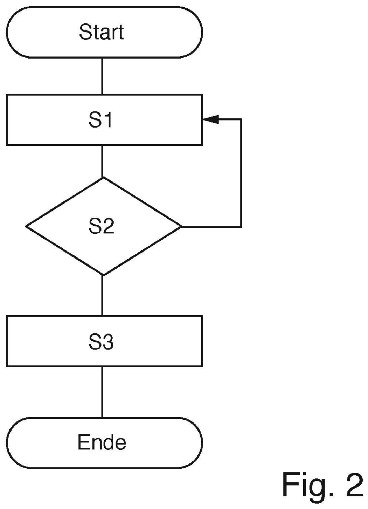

Die Steuereinrichtung bewirkt zunächst eine herkömmliche Hämodialyse, siehe in Fig. 2 Schritt S1, und eine anschließende eine Hämodiafiltration, siehe in Fig. 2 Schritt S3.

Sie Steuereinrichtung 5 beginnt mit der Hämodialyse im Schritt S1.

Dargestellt durch die Abfrage S2 überwacht die Steuereinrichtung 5 fortlaufend, ob sich eine Sekundärmembran auf einer Membran 1c des Dialysators 1 gebildet hat. Falls sich keine Sekundärmembran gebildet hat, wird die Hämodialyse fortgesetzt.

Sobald sich die Sekundärmembran auf der Membran 1c des Dialysators 1 gebildet hat, setzt die Steuereinrichtung 5 das Verfahren in Schritt S3 mit der Hämodiafiltration fort.

Während der Hämodiafiltration bewirkt die Steuereinrichtung 5 folgendes: Fördern von Blut durch eine Blutseite 1a des Dialysators 1 mittels der elektrischen Blutpumpe 2, Fördern von Substituat 3 mittels der elektrischen Substituatpumpe 4, und Einstellen eines Fördervolumenstroms der elektrischen Substituatpumpe 4 derart, dass eine Stromaufnahme der elektrischen Blutpumpe 2 innerhalb vorgebbarer Grenzwerte bleibt.

Bei einem Ansteigen der Stromaufnahme der elektrischen Blutpumpe 2 über einen oberen Grenzwert reduziert die Steuereinrichtung 5 den Fördervolumenstrom der elektrischen Substituatpumpe 4. Bei einem Abnehmen der Stromaufnahme der elektrischen Blutpumpe 2 unter einen unteren Grenzwert erhöht die Steuereinrichtung 5 den Fördervolumenstrom der elektrischen Substituatpumpe 4 (wieder).

Das Substituat 3 wird mittels einer Postdilution 9 einem Blutkreislauf 200 eines Patienten zugeführt.

Nach dem Umschalten auf die Hämodiafiltration stellt die Steuereinrichtung 5 eine berechnete Cross Rate ein und ermittelt eine sich ergebende Stromaufnahme der elektrischen Blutpumpe 2 bei der vorgegebenen Cross Rate. Der obere Grenzwert und der untere Grenzwert werden dann abhängig von der gemessenen Stromaufnahme ermittelt. Beispielsweise kann der obere Grenzwert in einem Bereich zwischen 115 % und 135 % der gemessenen Stromaufnahme vorgegeben werden und der untere Grenzwert kann als das 0,8-fache bis 0,95-fache des oberen Grenzwerts vorgegeben werden.

## Patentansprüche

1. Vorrichtung (100) zur extrakorporalen Blutbehandlung, mittels der eine Hämodialyse und eine Hämodiafiltration ausführbar ist, aufweisend:
- einen Dialysator (1),
- eine elektrische Blutpumpe (2) zum Fördern von Blut durch eine Blutseite (1a) des Dialysators (1),
- eine elektrische Substituatpumpe (4) zum Fördern von Substituat (3) und
- eine Steuereinrichtung (5), die die elektrische Blutpumpe (2) und die elektrische Substituatpumpe (4) derart ansteuert, dass während einer Hämodiafiltration
- Blut durch eine Blutseite (1a) des Dialysators (1) mittels der elektrischen Blutpumpe (2) gefördert wird,
- Substituat (3) mittels der elektrischen Substituatpumpe (4) gefördert wird, und
- ein Fördervolumenstrom der elektrischen Substituatpumpe (4) derart eingestellt wird, dass eine Stromaufnahme der elektrischen Blutpumpe (2) innerhalb vorgebbarer Grenzwerte bleibt.

2. Vorrichtung (100) zur extrakorporalen Blutbehandlung nach Anspruch 1, **dadurch gekennzeichnet, dass**
- die Steuereinrichtung (5) bei einem Ansteigen der Stromaufnahme der elektrischen Blutpumpe (2) über einen oberen Grenzwert den Fördervolumenstrom der elektrischen Substituatpumpe (4) reduziert und bei einem Abnehmen der Stromaufnahme der elektrischen Blutpumpe (2) unter einen unteren Grenzwert den Fördervolumenstrom der elektrischen Substituatpumpe (4) erhöht.

3. Vorrichtung (100) zur extrakorporalen Blutbehandlung nach einem der vorhergehenden Ansprüche, **dadurch gekennzeichnet, dass**
- das Substituat (3) mittels einer Postdilution einem Blutkreislauf (200) eines Patienten zugeführt wird.

4. Vorrichtung (100) zur extrakorporalen Blutbehandlung nach einem der vorhergehenden Ansprüche, **dadurch gekennzeichnet, dass**
- die Steuereinrichtung (5)
- zunächst eine Hämodialyse ausführt,
- fortlaufend überwacht, ob sich eine Sekundärmembran auf einer Membran (1c) des Dialysators (1) gebildet hat, und
- sobald sich die Sekundärmembran auf der Membran (1c) des Dialysators (1) gebildet hat, auf eine Hämodiafiltration umschaltet.

5. Vorrichtung (100) zur extrakorporalen Blutbehandlung nach Anspruch 4, **dadurch gekennzeichnet, dass**
- die Steuereinrichtung (5)
- eine vorgegebene Cross Rate nach dem Umschalten auf die Hämodiafiltration einstellt,
- eine sich ergebende Stromaufnahme der elektrischen Blutpumpe (2) bei der vorgegebenen Cross Rate misst und
- die vorgebbaren Grenzwerte abhängig von der gemessenen Stromaufnahme berechnet.

6. Vorrichtung (100) zur extrakorporalen Blutbehandlung nach Anspruch 5, **dadurch gekennzeichnet, dass**
- der obere Grenzwert in einem Bereich zwischen 115 % und 135 % der gemessenen Stromaufnahme vorgegeben wird, und/oder
- der untere Grenzwert als das 0,8-fache bis 0,95-fache des oberen Grenzwerts vorgegeben wird.
